# EUROPÄISCHE PATENTSCHRIFT

(11) **EP 3 531 985 B1**
(45) Veröffentlichungstag und Bekanntmachung des Hinweises auf die Patenterteilung: **24.02.2021**
(21) Anmeldenummer: 17803794.1
(22) Anmeldetag: 26.10.2017
(51) Int. Cl.: A61F 2/852, A61F 2/915

(54) **DOPPELSTENT**
DOUBLE STENT
STENT DOUBLE

(30) Priorität: 26.10.2016 DE 102016120445
(43) Veröffentlichungstag der Anmeldung: 04.09.2019
(73) Patentinhaber: MOB.Ing GmbH, 79539 Lörrach (DE)
(72) Erfinder: NEUSS, Malte, 53121 Bonn (DE)
(74) Vertreter: Schneiders & Behrendt Bochum
(86) Internationale Anmeldenummer: PCT/EP2017/077454
(87) Internationale Veröffentlichungsnummer: WO 2018/078019

(56) Entgegenhaltungen:
- WO-A2-2012/084202
- DE-U1- 29 623 983

## Beschreibung

Die Erfindung betrifft einen Doppelstent mit koaxial angeordneten Stents, wobei zwischen einem ersten inneren Stent und wenigstens zwei äußeren Stents eine erste Membran angeordnet ist. Der Doppelstent dient insbesondere als Stentgraft zur Überbrückung von vaskulären Fehlbildungen, etwa Aneurysmen und Shunts, aber auch zur Verstärkung von labilen, brüchigen oder thrombotischen Gefäßwänden. Er wird ferner eingesetzt als Überbrückung bei Abzweigungen aus gestenteten Gefäßen.

Stentgrafts zur Überbrückung von vaskulären Fehlbildungen sind in einer Vielzahl von Formen bekannt. In der Regel bestehen sie aus einem Stent, der mit einer Membran ganz oder teilweise überzogen ist. Die Membran schließt die vaskuläre Fehlbildung gegen das Gefäß ab, der Stent hält das Gefäß offen und sorgt dafür, dass die Membran eng an der Gefäßwand anliegt.

Ein Problem bei Stentgrafts ist die Verankerung der Membran am Stent. Hierzu wurden Doppelstents entwickelt, bei denen die Membran zwischen einem äußeren und einem inneren Stent gehalten wird. Bei der Expansion eines solchen Doppelstents nimmt die Membran an der radialen Erweiterung teil, bleibt aber zwischen den beiden Stents eingeklemmt.

Ein solcher Doppelstent ist beispielsweise aus der DE 197 20 115 A1 bekannt. Der dort beschriebene Stent hat sich an und für sich bewährt, ist aber in zweierlei Hinsicht verbesserungsfähig. DE 296 23 983 U1 offenbart einen Doppelstent mit den Merkmalen aus dem Oberbegriff des Anspruchs 1.

Zum einen ergeben sich häufig Probleme mit der Dichtigkeit, da die Membran nicht unmittelbar an der Gefäßwand anliegt und/oder bei der Expansion des Doppelstents beschädigt wird. In beiden Fällen wird der Doppelstent den an ihn gestellten Anforderungen, nämlich der Abschottung beispielsweise einer vaskulären Fehlbildung, nicht gerecht.

Zum anderen kann es bei der Expansion des Doppelstents dazu kommen, dass der Verbund aus zwei Stents und einer Membran an Zusammenhalt verliert, beispielsweise wenn die beiden Stents ein unterschiedliches Expansionsverhalten - etwa aufgrund örtlicher Gegebenheiten - haben.

Schließlich leidet bei derartigen Doppelstents die Manövrierfähigkeit. Zwei übereinander angeordnete Stents gleicher Länge führen zu einer Versteifung, die - insbesondere bei längeren Stents - zu Problemen bei der Passage durch und der Platzierung in gewundenen Gefäßen führt.

Bei der Verwendung normaler Stentgrafts reicht in zahlreichen Fällen die Radialkraft eines einzelnen Stents, sei er ballonexpandierbar oder selbst expandierend, nicht aus, um die Membran sicher zu verankern und/oder eine zuverlässige und dauerhafte Überbrückung oder Gefäßerweiterung sicherzustellen. In diesen Fällen wäre der Einsatz eines Doppelstents mit erhöhter Radialkraft sinnvoll. Dies gilt insbesondere für selbst expandierende Stents, die in der Regel eine geringere Radialkraft haben, als ballonexpandierbare Stents.

Der Erfindung liegt damit die Aufgabe zu Grunde, einen Doppelstent bereitzustellen, der zum einen den Anforderungen an die Dichtigkeit und Zuverlässigkeit genügt und zum anderen den notwendigen Zusammenhalt gewährleistet. Darüber hinaus soll der Stent eine hohe Radialkraft bei guter Flexibilität aufweisen.

Diese Aufgabe wird mit einem Doppelstent der eingangs genannten Art gelöst, bei dem auf den äußeren Stents eine zweite Membran angeordnet ist, wobei die Membranenden der ersten und der zweiten Membran an den Enden der Stents zusammengefasst sind, auf die Innenseite des ersten Stents umgeschlagen sind und unter Federzungen des ersten Stents festgeklemmt sind.

Der erfindungsgemäße Doppelstent weist nicht nur einen inneren und wenigstens zwei äußere Stents auf sondern auch eine innere und äußere Membran. Dabei ergänzen sich die inneren und äußeren Stents hinsichtlich der Radialkraft und die beiden Membranen hinsichtlich der Dichtigkeit. Die äußere zweite Membran dient als Schutz und Ergänzung der inneren ersten Membran; wenn die innere Membran bei der Expansion beschädigt wird, beispielsweise einreißt, ist die äußere Membran in der Lage, diesen Defekt auszugleichen und umgekehrt. Des Weiteren hält die äußere Membran das Konstrukt zusammen, wobei die Verankerung der Enden der äußeren Membran - zusammen mit den Enden der inneren Membran - auf der Innenseite des inneren Stent zu diesem Zusammenhalt beiträgt.

Für die erfindungsgemäß eingesetzten Stents kommen übliche Stentdesigns infrage, wie sie vielfach bei ballonexpandierbaren und selbst expandierenden Stents entwickelt wurden. Für ballonexpandierbare Stents kommen übliche Materialien in Frage, beispielsweise Stahllegierungen für den medizinischen Gebrauch, Kobalt-Chrom-Legierungen und dergleichen. Für selbst expandierende Stents kommen insbesondere Materialien mit einem Formgedächtnis infrage, etwa Nickel-Titan-Legierungen. Kombinationen von Stents aus diesen Materialien sind ebenfalls möglich. Weiterhin können die oder einzelne Stents aus Kunststoff bestehen. Eine Kombination von Kunststoff- und Metallstents sind ebenfalls möglich, wobei vorzugsweise der innere Stent aus Kunststoff besteht.

Besonders bevorzugt ist eine Kombination aus einem inneren Stent aus Nitinol oder einer anderen Formgedächtnislegierung und einem oder mehreren äußeren Stents aus einer Kobalt-Chrom-Legierung. Diese Kombination bietet Vorteile bei der Platzierung über einen Ballonkatheter, bezüglich der Radialkraft, die im Wesentlichen von den äußeren Stents bereitgestellt wird, und bezüglich der Offenhaltung des Gefäßes durch den inneren Stent.

Die Stents können geflochten sein, werden aber in der Regel aus einem Rohr mit geeignetem Durchmesser mithilfe eines Laserstrahls geschnitten. Sie verfügen über eine Maschenstruktur.

Die Stents können beispielsweise eine Maschenstruktur haben, wie sie von einander kreuzenden Stegen gebildet wird. Bevorzugt sind Stents, die aus einer Mehrzahl von mäandrierenden Ringsegmenten gebildet werden, wobei die Ringsegmente durch Verbindungsstege mit benachbarten Ringsegmenten verbunden sind. Auch in diesem Fall entstehen Maschen, deren Größe durch die Häufigkeit der Verbindungsstege zwischen zwei benachbarten Ringsegmenten bestimmt ist. Eine solche Stentstruktur ist geeignet, die bei der Expansion auftretende Längenreduktion zumindest teilweise auszugleichen, je nach Anordnung und Form der Verbindungsstege.

Die am inneren ersten Stent vorhandenen Federzungen können eine Vielzahl von Formen haben. Die Federzungen weisen dabei insbesondere stentauswärts, d.h., sie weisen zum Stentrand. Solche Federzungen sind insbesondere an Verbindungsstegen der Ringsegmente angeordnet, wobei die Folienenden zwischen den Verbindungsstegen und den von den Verbindungsstegen ausgehenden Federzungen festgeklemmt sind.

Vorzugsweise sind die Federzungen in die Verbindungsstege eingeschnitten, d.h. gegen die Verbindungsstege beweglich. Es ist ferner bevorzugt, die Federzungen an Verbindungsstegen anzuordnen, die im Randbereich des inneren Stents angeordnet sind, etwa das erste randständige Ringsegment mit dem zweiten, benachbarten verbinden.

Das Festklemmen der Membranenden auf der Innenseite des inneren Stent führt zu einer zuverlässigen Verankerung der beiden Membranen und stärkt den Verbund aus innerem Stent, innerer Membran, äußerem Stent und äußerer Membran.

Für die Membranen kann jedes dafür geeignete biologische oder künstliche Material verwandt werden. In der Regel bestehen die Membranen aus einem Kunststoff, vorzugsweise einem Kunststoffschlauch, der über den jeweiligen Stent gezogen ist. Ein geeignetes Material ist beispielsweise Polytetrafluorethylen, PTFE, insbesondere ePTFE, dass die benötigte Elastizität für die Expansion aufweist. Andere medizinisch geeignete Kunststoffe, etwa Polyester, Polyolefine, Polyurethane, Polyurethancarbonat und dergleichen, können ebenfalls eingesetzt werden.

Es versteht sich, dass für den inneren und die äußeren Stents unterschiedliche Designs verwandt werden können und die innere und äußere Membran aus verschiedenen Materialien gefertigt sein können.

Der innere und die - zusammengenommenen - äußeren Stents des erfindungsgemäßen Doppelstents haben vorzugsweise die gleiche Länge und eine 100%ige Überdeckung. Sie können aber auch gegeneinander versetzt - Teilüberdeckung - angeordnet sein oder unterschiedliche Länge aufweisen. Diese Varianten führen zu einer Versteifung des Deckungsbereiches und zu einer erhöhten Flexibilität in Bereichen fehlender Überdeckung, was zu einer guten Anpassungsfähigkeit bei gleichzeitig sicherem Sitz führt.

Das Doppelstentprinzip mit zwei Membranen führt naturgemäß zu einer relativ hohen Wandstärke des Konstrukts, was die Manövrierfähigkeit im Gefäßsystem eines Patienten einschränkt. Dem kann dadurch begegnet werden, dass die Wandstärke der zum Schneiden der Stents verwandten Rohre gering gewählt wird, beispielsweise im Bereich von 0,05 bis 0,50 mm, vorzugsweise 0,10 bis 0,20 mm und insbesondere etwa 0,15 mm. Auch die Stegbreite kann vermindert werden auf beispielsweise 0,05 bis 0,50 mm, vorzugsweise 0,10 bis 0,20 mm und insbesondere etwa 0,15 mm. Durch die Verwendung von zwei Stents wird dennoch eine hohe Radialkraft erreicht.

Es ist ferner bevorzugt, die äußeren Stents mit kleineren Maschen zu versehen als den inneren Stent. Hierdurch wird bei der Expansion eine Pressspannung erzeugt, die sich vorteilhaft auf die Radialkraft und den Zusammenhalt des Konstrukts auswirkt. Erzielt wird eine hohe Festigkeit und Dauerhaftigkeit des Konstrukts.

Es versteht sich, dass für die Platzierung des Doppelstents im Zielgefäß übliche Methoden zum Einsatz kommen, in der Regel ein Ballonkatheter, auf dem der Doppelstent aufgekrimpt wird.

Der erfindungsgemäße Doppelstent ist insbesondere geeignet, in Abzweige aus gestenteten Gefäßen eingesetzt zu werden und den sich dabei zwischen gestentetem Gefäß und Abzweigung bildenden Raum zu überbrücken.

In den erfindungsgemäßen Doppelstents können außen zwei oder mehr Stents verwandt werden, die jeder für sich eine Stützeinheit ausbilden. Neben zwei Stents, die beispielsweise an den Enden des Stents angeordnet sind, kann beispielsweise auch mittig ein dritter Stent angeordnet sein. Wesentlich ist, dass die äußeren Stents gegeneinander beweglich sind, so dass der Doppelstent in diesen Bereichen, in denen keine Überdeckung besteht oder die äußeren Stents aneinander stoßen, eine erhöhte Flexibilität aufweist. Dies erlaubt neben einer sehr guten Manövrierfähigkeit auch in kurvigen Gefäßen eine individuelle Anpassung der Stützfunktion an die Gegebenheiten eines Gefäßes.

Es versteht sich, dass die Dicke (Wandstärke) der äußeren Stents von der des inneren Stents abweichen kann. In diesem Fall weist in der Regel der innere Stent eine dickere Wandung auf als die äußeren.

Auch die äußeren Stents können eine unterschiedliche Dicke aufweisen, je nach Anforderung an die Flexibilität und Radialkraft.

In der Regel sind die hintereinander angeordneten äußeren Stents nicht miteinander verbunden. Eine lose Verbindung ist aber ebenfalls möglich, beispielsweise über Klebpunkte oder Schweißpunkte, Gelenkverbindungen oder eine kardanische Verbindung. Gelenkelemente zur Verbindung von Stents sind bekannt. Eine kardanische Verbindung entsteht beispielsweise durch die Verbindung zweier Stents mittels zweier Verbindungsstege, die einander gegenüber stehen können und die vorzugsweise auch gekrümmt oder s-förmig gebogen sind.

Für die Stents kommen die üblichen Materialien in Frage, beispielsweise medizinischer Stahl, Kobalt-Chrom-Legierungen und Nickel-Titan-Legierungen oder beliebige Kombinationen davon. Auch Kunststoffe, beispielsweise resorbierbare Kunststoffe, wie sie aus dem Stand der Technik bekannt sind, können eingesetzt werden, wie auch Kombinationen aus Metallstents und Kunststoffstents, etwa in Form eines inneren Metallstents und mehrerer äußerer Kunststoffstents.

Der Verbund eines inneren und mehrerer äußerer Stents mit einer zwischenliegenden und einer abdeckenden Membran, die auf der Innenseite des inneren Stents festgelegt ist, führt zu einem äußerst stabilen Implantat, das dennoch über eine hohe Flexibilität verfügt. Die beiden Membranen tragen zur Stabilisierung bei und erlauben es, die Stentwände sehr dünn zu halten. Dennoch verfügt der Stent über eine gute Radialkraft.

Die äußeren Stents können je nach Einsatzzweck angeordnet sein. Für eine verbesserte Stützfunktion in den Endbereichen reicht es aus, nur dort eine Überdeckung vorzunehmen. Ist eine Verstärkung über die gesamte Länge erforderlich, kann die äußere Stentlage aus einer Mehrzahl von Einzelstents bestehen, die isoliert sein können oder, wie oben beschrieben, auch verbunden sein können.

Die Erfindung wird durch die beiliegenden Abbildungen näher erläutert, die bevorzugte Ausführungsformen der Erfindung zeigen. Es versteht sich, dass in den Abbildungen gezeigten Merkmale jeweils für sich Teil der Erfindung sind und nicht nur im Zusammenhang mit anderen Merkmalen der Abbildung zu verstehen sind. Es zeigen:
- Figur 1: schematisch den Aufbau eines erfindungsgemäßen Stents;
- Figur 2: eine Variante des zum Einsatz kommenden Klemmprinzips der Membranen; und
- Figur 3: einen Schnitt durch die Wandung eines erfindungsgemäßen Stents.

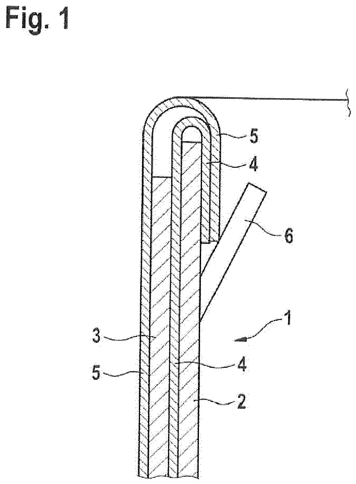

Der Doppelstent 1 gemäß Figur 1 besteht aus einem ersten inneren Stent 2 und einem zweiten äußeren Stent 3, die koaxial zueinander angeordnet sind. Der äußere Stent 3 ist um ein geringes kürzer als der innere Stent 2. Der Doppelstent ist im nicht expandierten Zustand dargestellt. Zwischen dem inneren Stent 2 und dem äußeren Stent 3 befindet sich eine erste innere Membran 4, auf dem äußeren Stent 3 eine zweite äußere Membran 5. Beide Membranen bestehen aus ePTFE.

Die innere Membran 4 und die äußere Membran 5 werden an ihren beiden Enden jeweils zusammengefasst und um den Rand der beiden Stents nach innen in den Hohlraum des inneren Stents 2 umgeschlagen. Schematisch dargestellt ist eine von mehreren Federzungen 6, die nach innen aufgebogen ist und unter die die Membranenden ragen. Die Federzungen 6 werden zur Festlegung der Membranenden nach außen zurückgebogen, so dass die Membranenden unter ihnen festgeklemmt sind.

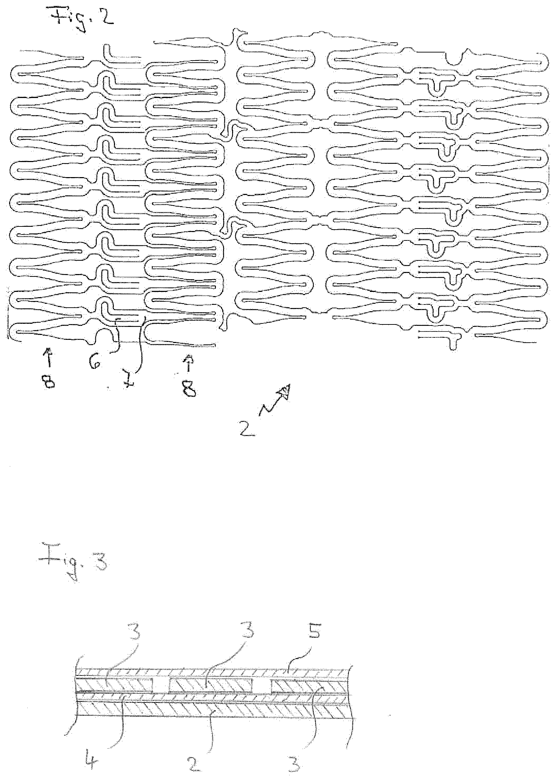

Figur 2 zeigt ein Stentdesign für den inneren Stent 2, bei dem zwei Ringsegmente 8, die benachbart sind und in der Nähe zum Rand angeordnet sind, durch Verbindungsstege 7 miteinander verbunden sind. In die Verbindungsstege 7 sind Federzungen 6 eingeschnitten, die gegen die Stentebene beweglich sind.

Die Federzungen 7 sind, zur Vergrößerung der Kontaktfläche zur Schlauchfolie (nicht dargestellt), im freien Endbereich abgewinkelt, was die Klemmwirkung in Bezug auf die eingeschobene Membran verbessert.

Figur 3 zeigt das Prinzip des erfindungsgemäßen Doppelstents mit einem einzelnen inneren Stent 2, zwei endständigen Stents 3 sowie einen mittelständigen Stent 3 sowie der zwischenliegenden Membran 4 und der Deckmembran 5, die beide nach innen umgeschlagen sind.

## Patentansprüche

1. Doppelstent mit koaxial angeordneten Stents, wobei zwischen einem ersten inneren Stent (2) und wenigstens zwei äußeren Stents (3) eine erste Membran (4) angeordnet ist, **dadurch gekennzeichnet, dass** auf den äußeren Stents (3) eine zweite Membran (5) angeordnet ist und dass die Membranenden der ersten (4) und der zweiten (5) Membran an den Enden der Stents (3, 4) zusammengefasst sind, auf die Innenseite des erstens Stents (3) umgeschlagen und unter Federzungen (6) des ersten Stents (3) festgeklemmt sind.

2. Doppelstent nach Anspruch 1, **dadurch gekennzeichnet, dass** wenigstens einer der Stents (2, 3) eine Maschenstruktur aufweist.

3. Doppelstent nach Anspruch 1 oder 2, **dadurch gekennzeichnet, dass** wenigstens der Stent (2) eine Mehrzahl von nebeneinander angeordneten Ringsegmenten (8) mit mäandrierender Struktur aufweist, die durch Stege (7) miteinander verbunden sind.

4. Doppelstent nach Anspruch 3, **dadurch gekennzeichnet, dass** die Federzungen (6) an Verbindungsstegen (7) zwischen nebeneinander angeordneten Ringsegmenten (8) des ersten Stents (2) angeordnet sind.

5. Doppelstent nach Anspruch 4, **dadurch gekennzeichnet, dass** die Federzungen (6) in die Verbindungsstege (7) eingeschnitten sind.

6. Doppelstent nach einem der vorstehenden Ansprüche, **dadurch gekennzeichnet, dass** die Federzungen (6) stentauswärts weisen.

7. Doppelstent nach einem der vorstehenden Ansprüche, **dadurch gekennzeichnet, dass** die Federzungen (6) in den Randbereichen des ersten Stents (2) angeordnet sind, vorzugsweise in Verbindungsstegen (7) zwischen dem ersten und zweiten randständigen Ringsegment (8) des ersten Stents (2).

8. Doppelstent nach einem der vorstehenden Ansprüche, **gekennzeichnet durch** zwei äußere Stents (3).

9. Doppelstent nach Anspruch 8, **dadurch gekennzeichnet, dass** die beiden äußeren Stents (3) an den Enden des Doppelstents angeordnet sind.

10. Doppelstent nach Anspruch 8, **dadurch gekennzeichnet, dass** die beiden äußeren Stents (3) flexibel miteinander verbunden sind.

11. Doppelstent nach einem der vorstehenden Ansprüche, **dadurch gekennzeichnet, dass** der zweite Stent (3) eine dichtere Struktur aufweist als der erste Stent (2).

12. Doppelstent nach einem der vorstehenden Ansprüche, **dadurch gekennzeichnet, dass** die Stege des ersten (2) und zweiten (3) Stents auf Lücke stehen.

13. Doppelstent nach einem der vorstehenden Ansprüche, **dadurch gekennzeichnet, dass** die erste und/oder zweite Membran (4, 5) aus einem Kunststoff besteht.

14. Doppelstent nach Anspruch 10, **dadurch gekennzeichnet, dass** die erste und/oder zweite Membran (4, 5) aus ePTFE besteht.

## Claims

1. Double stent comprising coaxially arranged stents, wherein a first membrane (4) is arranged between a first inner stent (2) and at least two outer stents (3), **characterised in that** a second membrane (5) is arranged on the outer stents (3), and **in that** the membrane ends of the first (4) and second (5) membranes are brought together at the ends of the stents (3, 4), are folded over onto the inner face of the first stent (3) and are clamped under spring tongues (6) of the first stent (3).

2. Double stent according to claim 1, **characterised in that** at least one of the stents (2, 3) has a mesh structure.

3. Double stent according to claim 1 or 2, **characterised in that** at least the stent (2) comprises a plurality of adjacently arranged annular segments (8) that have a meandering structure and are interconnected by webs (7).

4. Double stent according to claim 3, **characterised in that** the spring tongues (6) are arranged on connecting webs (7) between adjacently arranged annular segments (8) of the first stent (2).

5. Double stent according to claim 4, **characterised in that** the spring tongues (6) are cut into the connecting webs (7).

6. Double stent according to any of the preceding claims, **characterised in that** the spring tongues (6) point outwards from the stent.

7. Double stent according to any of the preceding claims, **characterised in that** the spring tongues (6) are arranged in the edge regions of the first stent (2), preferably in connecting webs (7) between the first and second annular edge segment (8) of the first stent (2).

8. Double stent according to any of the preceding claims, **characterised by** two outer stents (3).

9. Double stent according to claim 8, **characterised in that** the two outer stents (3) are arranged on the ends of the double stent.

10. Double stent according to claim 8, **characterised in that** the two outer stents (3) are flexibly interconnected.

11. Double stent according to any of the preceding claims, **characterised in that** the second stent (3) has a denser structure than the first stent (2).

12. Double stent according to any of the preceding claims, **characterised in that** the webs of the first (2) and second (3) stent are staggered.

13. Double stent according to any of the preceding claims, **characterised in that** the first and/or second membrane (4, 5) consists of a plastics material.

14. Double stent according to claim 10, **characterised in that** the first and/or second membrane (4, 5) consists of ePTFE.

## Revendications

1. Stent double avec des stents disposés de manière coaxiale, dans lequel une première membrane (4) est disposée entre un premier stent intérieur (2) et au moins deux stents extérieurs (3), **caractérisé en ce qu'**une deuxième membrane (5) est disposée sur les stents extérieurs (3), et que les extrémités de membrane de la première (4) et de la deuxième (5) membrane sont regroupées au niveau des extrémités des stents (3, 4), sont retournées sur le côté intérieur du premier stent (3) et sont coincées sous des languettes élastiques (6) du premier stent (3).

2. Stent double selon la revendication 1, **caractérisé en ce qu'**au moins un des stents (2, 3) présente une structure à mailles.

3. Stent double selon la revendication 1 ou 2, **caractérisé en ce qu'**au moins le stent (2) présente une multitude de segments annulaires (8) disposés côte à côte avec une structure en méandre, qui sont reliés les uns aux autres par des entretoises (7).

4. Stent double selon la revendication 3, caractérisé ne ce que les languettes élastiques (6) sont disposées au niveau d'entretoises de liaison (7) entre des segments annulaires (8) disposés côte à côte du premier stent (2).

5. Stent double selon la revendication 4, **caractérisé en ce que** les languettes élastiques (6) sont entaillées dans les entretoises de liaison (7).

6. Stent double selon l'une quelconque des revendications précédentes, **caractérisé en ce que** les languettes élastiques (6) pointent vers l'extérieur du stent.

7. Stent double selon l'une quelconque des revendications précédentes, **caractérisé en ce que** les languettes élastiques (6) sont disposées dans les zones de bord du premier stent (2), de préférence dans des entretoises de liaison (7) entre le premier et le deuxième segment annulaire (8) marginal du premier stent (2).

8. Stent double selon l'une quelconque des revendications précédentes, **caractérisé par** deux stents extérieurs (3).

9. Stent double selon la revendication 8, **caractérisé en ce que** les deux stents extérieurs (3) sont disposés au niveau des extrémités du stent double.

10. Stent double selon la revendication 8, **caractérisé en ce que** les deux stents extérieurs (3) sont reliés l'un à l'autre de manière flexible.

11. Stent double selon l'une quelconque des revendications précédentes, **caractérisé en ce que** le second stent (3) présente une structure plus dense que le premier stent (2).

12. Stent double selon l'une quelconque des revendications précédentes, **caractérisé en ce que** les entretoises du premier (2) et du second stent (3) sont disposées en quinconce.

13. Stent double selon l'une quelconque des revendications précédentes, **caractérisé en ce que** la première et/ou la deuxième membrane (4, 5) consistent en une matière plastique.

14. Stent double selon la revendication 10, **caractérisé en ce que** la première et/ou la deuxième membrane (4, 5) consistent en du ePTFE.
